# EUROPÄISCHE PATENTSCHRIFT

(11) **EP 0 553 778 B1**
(45) Veröffentlichungstag und Bekanntmachung des Hinweises auf die Patenterteilung: **12.12.2001**
(21) Anmeldenummer: 93101179.5
(22) Anmeldetag: 27.01.1993
(51) Int. Cl.: C07D 401/06, C07F 9/50, C07F 15/00

(54) **Asymmetrische Hydrierung**
Asymmetric hydrogenation
Hydrogénation asymmétrique

(30) Priorität: 31.01.1992 CH 28992
(43) Veröffentlichungstag der Anmeldung: 04.08.1993
(73) Patentinhaber: F. HOFFMANN-LA ROCHE AG, 4002 Basel (CH)
(72) Erfinder: Broger, Emil Albin, CH-4312 Magden (CH); Hofheinz, Werner, CH-4103 Bottmingen (CH); Meili, Arthur, CH-4125 Riehen (CH)
(74) Vertreter: Braun, Axel

(56) Entgegenhaltungen:
- EP-A- 0 026 894
- CHEMICAL ABSTRACTS, vol. 116, 1992, Columbus, Ohio, US; abstract no. 20725g, SAKURABA, SHUNJI 'Asymmetric reactions catalyzed by chiral metal complexes.' Seite 544 & Synlett 1991, (10), pages 689-690
- D. ARNTZ AND A. SCHÄFER 'Catal. Met. Complexes, 12' 1991 , KLUWER ACADEMIC PUBLISHERS

## Beschreibung

Die vorliegende Erfindung betrifft ein neues Verfahren zur Herstellung von (R)- bzw. (S)-Enantiomeren der Verbindung der Formel

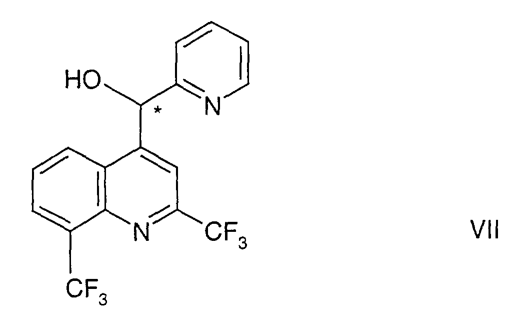

Dieses Verfahren ist dadurch gekennzeichnet, dass man das Keton der Formel 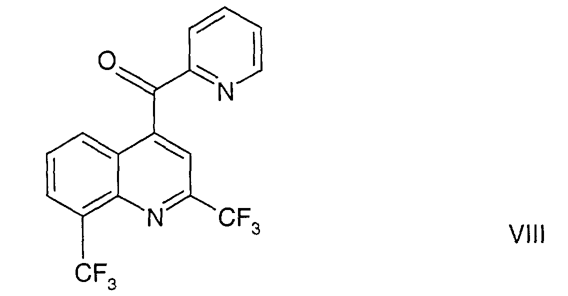 asymmetrisch hydriert, und zwar in Gegenwart eines Rhodium-Diphosphin-Komplexes der Formel

[Rh(X)(Y)(L_{0,1,2}]_{1,2} III

worin X, Y und L folgende Bedeutung haben:
- X=: Halogenid, Z-COO-, Phenolat oder halogeniertes Phenolat;
- Z =: C₁-C₄-Alkyl, unsubstituiertes Phenyl, halogeniertes C₁-C₄-Alkyl oder halogeniertes Phenyl;
- Y =: chiraler, atropisomerer Diphosphinligand der Formel 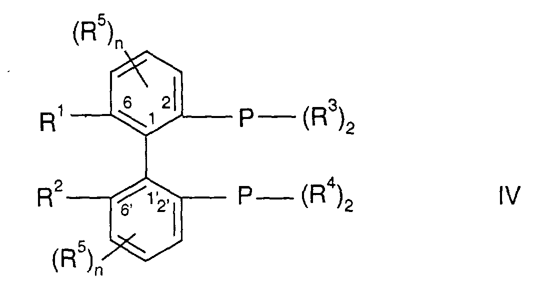 oder der Formel 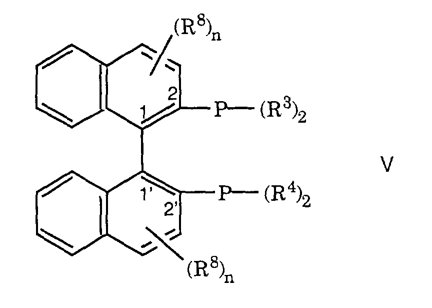 oder ein Ferrocenyl-Diphosphin der Formel 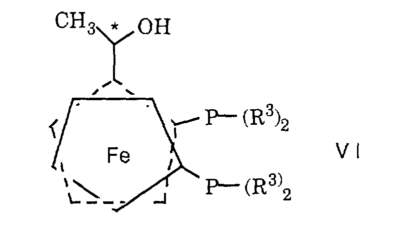 mit:
- R¹,R² =: unabhängig voneinander C₁-C₄-Alkyl, C₁-C₄-Alkoxy, Di-C₁-C₄-Alkylamino, Hydroxy, durch Benzyl, Allyl, Benzyloxymethyl, C₁-C₄-Alkoxymethyl oder 2-Methoxyäthoxymethyl geschütztes Hydroxy oder Hydroxymethyl, Hydroxymethyl, oder R¹ und R² zusammen die Gruppen

(―CH₂―)ₘ, ―CH₂―O―CH₂―, 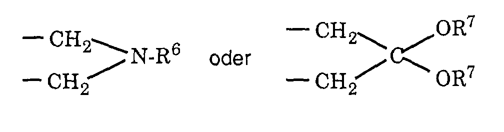
- m=: Zahl 3, 4, 5,
- R⁶ =: C₁-C₄-Alkyl oder unsubstituiertes oder einfach oder mehrfach durch Phenyl, C₁-C₄-Alkyl, C₁-C₄-Alkoxy, di-C₁-C₄-Alkylamino, Fluor oder Tri-C₁-C₄-Alkylsilil substituiertes Phenyl oder Benzyl,
- R⁷ =: C₁-C₄-Alkyl oder beide R⁷ zusammen Di- oder Trimethylen,
- R³,R⁴ =: unabhängig voneinander C₁-C₄-Alkyl, oder unsubstituiertes oder einfach oder mehrfach durch Phenyl, C₁-C₄-Alkyl, C₁-C₄-Alkoxy, di-C₁-C₄-Alkylamino, Fluor oder Tri-C₁-C₄-Alkylsilil substituiertes Phenyl, C₃-C₇-Cycloalkyl, einen fünfgliedrigen Heteroaromaten der Formeln (a), (b), (c), oder (d) 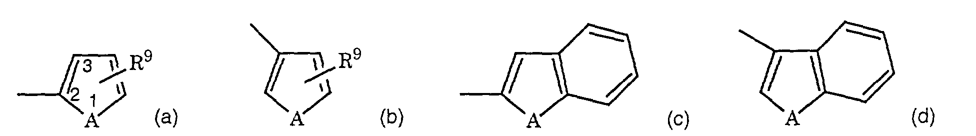 worin A Sauerstoff, Schwefel oder NR¹⁰, R⁹ Wasserstoff, C₁-C₄-Alkyl, C₁-C₄-Alkoxy und R¹⁰ C₁-C₄-Alkyl, bedeutet
oder eine Gruppe der Formel 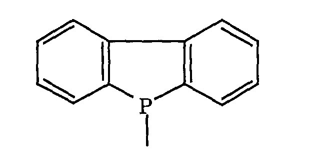
- R⁵ =: C₁-C₄-Alkyl, C₁-C₄-Alkoxy,
- R⁸ =: bevorzugt in 5,5'-Position, Methyl, Aethyl, Halogen, -OH, -NH₂, Acetylamino, -NO₂, -SO₃H,
- n=: Zahl 0, 1, 2, 3, und
- L=: neutraler Ligand, ausgewählt aus einer Gruppe von Olefinen, bestehend aus Ethylen, Propylen, Cycloocten, 1,5-Hexadien, Norbornadien, 1,5-Cyclooctadien oder von Nitrilen, bestehend aus Acetonitril oder Benzonitril oder von den verwendeten Lösungsmitteln.

Der Ausdruck C₁-C₄-Alkyl" bedeutet, im Rahmen der vorliegenden Erfindung, geradkettige oder verzweigte Alkylgruppen mit 1 bis 4 Kohlenstoffatomen wie Methyl, Aethyl, Propyl, Isopropyl, n-Butyl, Isobutyl und tert. Butyl. Der Ausdruck C₁-C₄-Alkoxy" bedeutet Gruppen, in denen der Alkylrest die vorhergehende Bedeutung hat.

In den Substituenten des fünfgliedrigen Heteroaromaten der Formeln (a) bis (d) bedeutet der Substituent R⁹ hier Wasserstoff, C₁-C₄-Alkyl, insbesondere Methyl, oder C₁-C₄-Alkoxy, insbesondere Methoxy, und R¹⁰ steht für C₁-C₄-Alkyl, vorzugsweise Methyl.

Die erwähnten Phenyl- und Benzylreste sind, im Rahmen der vorliegenden Erfindung, sowohl unsubstituiert als auch in ortho, meta oder para Stellung oder auch mehrfach substituiert. Substituenten sind Phenyl, C₁-C₄-Alkyl- oder C₁-C₄-Alkoxygruppen, vorzugsweise Methyl- oder Methoxygruppen, oder auch Di-C₁-C₄-Alkylamino, vorzugsweise Dimethylaminogruppen, sowie Fluor oder auch Tri-C₁-C₄-Alkylsilyl wie Trimethylsilyl und dergleichen.

Der Ausdruck "Cycloalkyl" bedeutet hier drei- bis siebengliedrige Ringe wie Cyclopropyl, Cyclobutyl, Cyclopentyl, Cyclohexyl und Cycloheptyl, insbesondere Cyclopentyl und Cyclohexyl.

Der Ausdruck "Halogen" bedeutet Fluor, Chlor, Brom oder Jod.

Der neutrale Ligand L kann bei der Hydrierung ausgetauscht werden. Falls mehr als ein solcher Ligand vorhanden ist, können diese auch voneinander verschieden sein.

Die asymmetrischen Hydrierungen können in geeigneten, unter den Reaktionsbedingungen inerten organischen Lösungsmitteln durchgeführt werden. Als solche können insbesondere genannt werden: aromatische Kohlenwasserstoffe wie Benzol oder Toluol, cyclische Aether wie Tetrahydrofuran oder Dioxan, Ester wie z.B. Essigester oder auch Gemische hiervon und dergleichen. Das Verhältnis zwischen Rhodium und dem Liganden Y liegt zweckmässig zwischen etwa 0,05 und etwa 5 Mol, vorzugsweise zwischen etwa 0,5 und etwa 2 Mol Rhodium pro Mol Ligand. Das Verhältnis zwischen Rhodium und dem Rest X liegt zweckmässig zwischen etwa 0,01 und etwa 20, vorzugsweise zwischen etwa 0,5 und etwa 10 Mol Rhodium pro Mol Rest X. Das molare Verhältnis zwischen Rhodium in den Komplexen der Formel III und den zu hydrierenden Verbindungen II liegt zweckmässig zwischen etwa 0,001 und etwa 5 Mol.%, vorzugsweise zwischen etwa 0,005 und etwa 0,2 Mol.%.

Die asymmetrischen Hydrierungen unter Verwendung der Komplexe der Formel III können vorzugsweise bei Temperaturen von etwa 20°C bis etwa 140°C, insbesondere von etwa 60°C bis etwa 120°C, durchgeführt werden. Diese Hydrierungen erfolgen auch zweckmässig unter Druck, insbesondere unter einem Druck von etwa 1 bis 100 bar, vorzugsweise 2 bis 60 bar.

Die Liganden der Formeln IV, V und VI sind bekannte Verbindungen bzw. Analoge bekannter Verbindungen, welche leicht in zur Herstellung von bekannten Liganden analoger Weise hergestellt werden können.

Diejenigen Verbindungen der Formeln IV und V, worin R³ und R⁴ gleich sind, können beispielsweise analog zu EP 104 375 oder auch EP 398 132 erhalten werden.

Diejenigen Verbindungen, worin R³ und R⁴ voneinander verschieden sind, können analog hierzu, jedoch in zwei Stufen, erhalten werden, z.B. gemäss folgendem Formelschema: 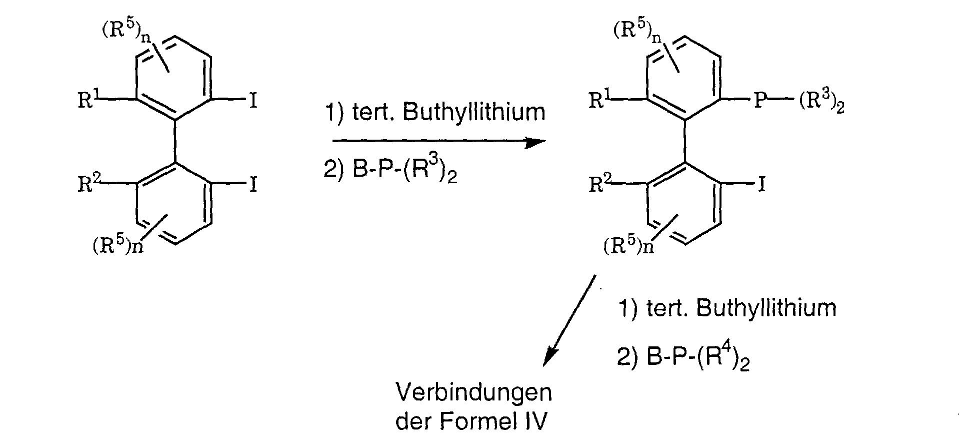
R¹, R², R³, R⁴, R⁵, n haben vorherige Bedeutung,
B = austretende Gruppe wie z.B. Halogen, insbesondere Chlor oder Brom, sowie Alkoxygruppen wie Methoxy und dergleichen.

Um zu gewährleisten, dass jeweils nur ein Jod durch Lithium ersetzt wird, werden die entsprechenden Reaktionen zweckmässig mit etwa äquimolaren Mengen an Reaktionspartnern durchgeführt.

Die Komplexe der Formel III können in an sich bekannter Weise hergestellt werden, z.B. indem man eine Verbindung der Formeln IV, V oder VI mit einer Verbindung, welche Rhodium abgeben kann, in einem geeigneten, inerten organischen oder wässrigen Lösungsmittel umsetzt. Als geeignete, Rhodium abgebende Verbindungen können beispielsweise genannt werden: organische Rhodium-Komplexe mit Aethylen, Propylen und dergleichen, sowie mit bis-Olefinen, z.B. (Z,Z)-1,5-Cyclooctadien, 1,5-Hexadien, Bicyclo[2.2.1]hepta-2,5-dien oder mit weiteren Dienen, welche mit Rhodium leicht lösliche Komplexe bilden. Bevorzugte, Rhodium abgebende Verbindungen sind z.B. Di-µ-chloro-bis[η⁴-(Z,Z)-1,5-cyclooctadien]dirhodium(I), Di-µ-chloro-bis[η⁴-norbomadien]dirhodium(I), Di-µ-trifluoracetato-bis[η⁴-(Z,Z)-1,5-cyclooctadien]dirhodium(I), Bis[η⁴-(Z,Z)-1,5-cyclooctadien]rhodium-tetrafluorborat oder Bis[η⁴-(Z,Z)-cycloctadien]rhodium-perchlorat.

Von den im Rahmen der vorliegenden Erfindung bevorzugt verwendeten Liganden Y, sind diejenigen der Formeln IV und VI, insbesondere diejenigen der Formel IV, bevorzugt.

Eine bevorzugte Ausführungsform des erfindungsgemässen Verfahrens betrifft die Verwendung eines Katalysators der Formel III, worin
X Chlor oder Brom, vorzugsweise Brom, bedeutet und Y einen chiralen, atropisomeren Liganden der Formel IV bedeutet, worin R¹ und R² gleich sind und Methyl oder Methoxy darstellen, n = 0 ist und R³ und R⁴ voneinander verschieden sind und Phenyl bzw. Cyclohexyl darstellen.

Die Verbindung der Formel VIII, sowie die (R)- und (S)-Verbindungen der Formel VII sind bekannte Verbindungen. Letztere sind Zwischenprodukte zur Herstellung der enantiomerenreinen erythro- bzw. threo-Isomeren der Formel

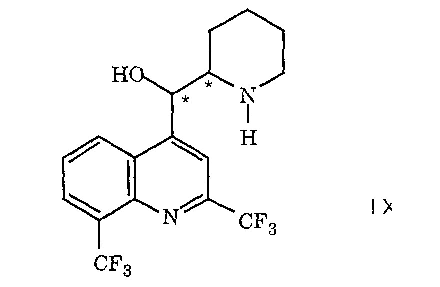

Die hier in Frage stehende Hydrierung des Pyridinringes, kann in an sich bekannter Weise, z.B. in Gegenwart eines Pt-Katalysators, durchgeführt werden, wobei überraschenderweise keine Racemisierung am die Hydroxygruppe tragenden C-Atom auftritt.

Die racemische erythro-Form der Verbindung der Formel IX ist, unter der Bezeichnung Mefloquin, ein bekanntes Antimalaria-Mittel.

Das erfindungsgemässe Verfahren erlaubt erstmals einen wirtschaftlichen Zugang zu den optisch reinen Isomeren der Verbindung der Formel IX, insbesondere von Mefloquin.

Die folgenden Beispiele dienen zur Erläuterung der Erfindung und stellen keinerlei Einschränkung hiervon dar. In diesen Beispielen haben die verwendeten Abkürzungen folgende Bedeutung:
- DC: Dünnschichtchromatographie
- GC: Kapillar-Gaschromatographie
- e.e.: Enantiomeric Excess. Der e.e. der Hydrierprodukte wurde durch GC an einer permethylierten Cyclodextrinphase bestimmt.
- BPPFOH: (1R)-1,1'-Bis(diphenylphosphino)-2-[(S)-1-hydroxyethyl]ferrocen
- BIPHEMP: (6,6'-Dimethylbiphenyl-2,2'-diyl)bis(diphenylphosphin)
- Cy₂BIPHEMP: P,P-Dicyclohexyl-P',P'-diphenyl-(6,6'-dimethylbiphenyl-2,2'-diyl)diphosphin
- Cy₄BIPHEMP: (6,6'-Dimethylbiphenyl-2,2'-diyl)bis(dicyclohexylphosphin)
- 2-Thienyl₂BIPHEMP: P,P-Diphenyl-P',P'-di-2-thienyl-(6,6'-dimethylbiphenyl-2,2'-diyl)diphosphin
- HOBIPHEP: (6,6'-Dihydroxybiphenyl-2,2'-diyl)bis(diphenylphosphin)
- MeOBIPHEP: (6,6'-Dimethoxybiphenyl-2,2'-diyl)bis(diphenylphosphin)
- (3,4,5-TriMeO)-MeOBIPHEP: (6,6'-Dimethoxybiphenyl-2,2'-diyl)bis(di-3,4,5-trimethoxyphenyl)phosphin)
- Cy₂pTolBIPHEMP: P,P-Dicyclohexyl-P',P'-di-p-tolyl-(6,6'-dimethylbiphenyl-2,2'-diyl)diphosphin
- Cy₂MeOBIPHEP: P,P-Dicyclohexyl-P',P'-diphenyl-(6,6'-dimethoxybiphenyl-2,2'-diyl)diphosphin
- Cyp₂MeOBIPHEP: P,P-Dicyclopentyl-P',P'-diphenyl-(6,6'-dimethoxybiphenyl-2,2'-diyl)diphosphin
- Ipr₂MeOBIPHEP: P,P-Diisopropyl,P',P',diphenyl-(6,6'-dimethoxybiphenyl-2,2'-diyl)diphosphin

Die Temperaturen sind in Grad Celsius angegeben.

### Beispiel 1

a) In einer Glove-Box (O₂-Gehalt <1 ppm) wurden in einem 50 ml-Glaskolben 6,2 mg (0,0125 mMol) Di-µ-chloro-bis-(1,5-Cyclooctadien)dirhodium(I) und 15,0 mg (0,025 mMol) (R,S)(-)-BPPFOH in 20 ml Aethylacetat suspendiert. Die Suspension wurde anschliessend 5 Minuten gerührt, wobei sich eine hellorange, klare Lösung bildete.
b) In einer Glove-Box (O₂-Gehalt <1 ppm) wurde ein 500 ml-Autoklav mit 1,85 g (5,0 mMol) 2-Pyridyl-2,8-bis(trifluormethyl)-4-chinolylketon, mit der oben hergestellten Katalysatorlösung und mit 130 ml Aethylacetat beladen. Die Hydrierung wurde bei 60°, einem konstanten Druck von 60 bar reinem H₂ und unter intensivem Rühren durchgeführt. Nach 19 Stunden Hydrierzeit betrug der Umsatz gemäss GC 84,2%. Die gelbe Hydrierlösung wurde am Rotationsverdampfer bei 45°/20 mbar eingedampft. Der Rückstand (1,86 g) wurde in 5 ml CH₂Cl₂ gelöst und zur Abtrennung des Katalysators durch eine Kieselgelsäule (Durchmesser 2 cm, Länge 2 cm) filtriert. Das Produkt wurde mit 100 ml CH₂Cl₂ eluiert. Das leicht gelbe Filtrat wurde am Rotationsverdampfer bei 30°/500 mbar eingedampft. Man erhielt 1,68 g (90,3%) gelbe Kristalle, die 84% (S)-α-(2-Pyridyl)-2,8-bis(trifluormethyl)-4-chinolinmethanol von 82,4% e.e. enthielten.

### Beispiel 2

a) In einer Glove-Box (O₂-Gehalt <1 ppm) wurden in einem 50 ml-Glaskolben 10,2 mg (0,025 mMol) Bis-(1,5-Cyclooctadien)rhodium(I)-tetrafluoroborat, 14,1 mg (0,025 mMol) (R)-Cy₂BIPHEMP und 8,1 mg (0,025 mMol) Tetrabutylammoniumbromid in 20 ml Toluol suspendiert. Die Suspension wurde anschliessend 60 Minuten gerührt, wobei sich eine orange, klare Lösung bildete.
b) In einer Glove-Box (O₂-Gehalt <1 ppm) wurde ein 500 ml-Autoklav mit 14,8 g (40 mMol) 2-Pyridyl-2,8-bis(trifluormethyl)-4-chinolylketon, mit der oben hergestellten Katalysatorlösung und mit 134 ml Toluol beladen. Die Hydrierung wurde bei 60°, einem konstanten Druck von 60 bar H₂ und unter intensivem Rühren durchgeführt. Nach 19 Stunden Hydrierzeit betrug der Umsatz 100% (DC). Eine analog Beispiel 1b) aufgearbeitete Probe (0,6 g) hatte einen e.e.-Wert von 90,4%. Die restliche Hydrierlösung wurde am Rotationsverdampfer bei 45°/20 mbar eingedampft. Zur Kristallisation des Produktes wurde der Rückstand (13,8 g) in 21 ml Toluol heiss gelöst, langsam auf Raumtemperatur abgekühlt und 16 Stunden gerührt. Durch Abkühlen auf 1° und Rühren während 3 Stunden wurde die Kristallisation vervollständigt. Die gelben Kristalle wurden abfiltriert, mit kaltem Toluol gewaschen und während 18 Stunden bei 50°/20 mbar getrocknet. Man erhielt 12,7 g (92%) (R)-α-(2-Pyridyl)-2,8-bis(trifluormethyl)-4-chinolinmethanol; Smp.: 130-131°; DC 1 Fleck; 91,8% e.e.; [α]_{D},²⁰ +17,1° (c=1, MeOH).

Zweimalige Umkristallisation aus Aethanol/Wasser lieferte reines (R)-Enantiomeres [(S)-Isomeres im GC nicht nachweisbar]; [α]_{D},²⁰ +18,7° (c=1, MeOH), Smp. 136,5-137,7°.

Auf analoge Weise wurde hergestellt:
(S)-α-(2-Pyridyl)-2,8-bis(trifluormethyl)-4-chinolinmethanol; Smp. 136-137°; [α]_{D},²⁰ -17,8° (c=1, MeOH).

### Beispiel 3

a) In einer Glove-Box (O₂-Gehalt 1 ppm) wurden in einem 20 ml-Messkolben 30,5 mg (0,25 mMol) Benzoesäure und 64,9 mg (0,25 mMol) Tetrabutylammoniumhydroxid in Toluol gelöst und die Lösung auf ein Volumen von genau 20,0 ml eingestellt. Die klare, farblose Lösung wurde während 5 Minuten gerührt. In einem 50 ml-Glaskolben wurden 10,2 mg (0,025 mMol) Bis-(1,5-Cyclooctadien)rhodium(I)-tetrafluorborat, 14,1 mg (0,025 mMol) (R)-Cy₂BIPHEMP und 2 ml (0,025 mMol) der oben hergestellten Tetrabutylammoniumbenzoat-Lösung zu 20 ml Toluol gegeben. Die Suspension wurde anschliessend 5 Minuten gerührt, wobei sich eine orange, klare Lösung bildete.
b) In einer Glove-Box (O₂-Gehalt <1 ppm) wurde ein 500 ml-Autoklav mit 14,8 g (40 mMol) 2-Pyridyl-2,8-bis(trifluormethyl)-4-chinolylketon, mit der oben hergestellten Katalysatorlösung und mit 132 ml Toluol beladen. Die Hydrierung wurde bei 60°, einem konstanten Druck von 60 bar H₂ und unter intensivem Rühren durchgeführt. Nach 19 Stunden Hydrierzeit betrug der Umsatz gemäss GC 84,3%. Die orange Hydrierlösung wurde am Rotationsverdampfer bei 45°/20 mbar eingedampft. Man erhielt 14,6 g (98%) braune Kristalle, die 84% (R)-α-(2-Pyridyl)-2,8-bis(trifluormethyl)-4-chinolinmethanol enthielten. Eine analog Beispiel 1b) aufgearbeitete Probe (0,6 g) hatte einen e.e. von 81,9%.

### Beispiele 4-22

In zu den Beispielen 1-3 analoger Weise wurden die in Tabelle 1 aufgeführten Hydrierungen durchgeführt, und zwar mit einem Komplex der Formel

[Rh(X)(Y)]

- S/C =: Molares Substrat-Katalysator-Verhältnis
- LM =: Lösungsmittel
- c =: Substratkonzentration im Hydriergemisch
- C₆F₅O =: Pentafluor-phenolat

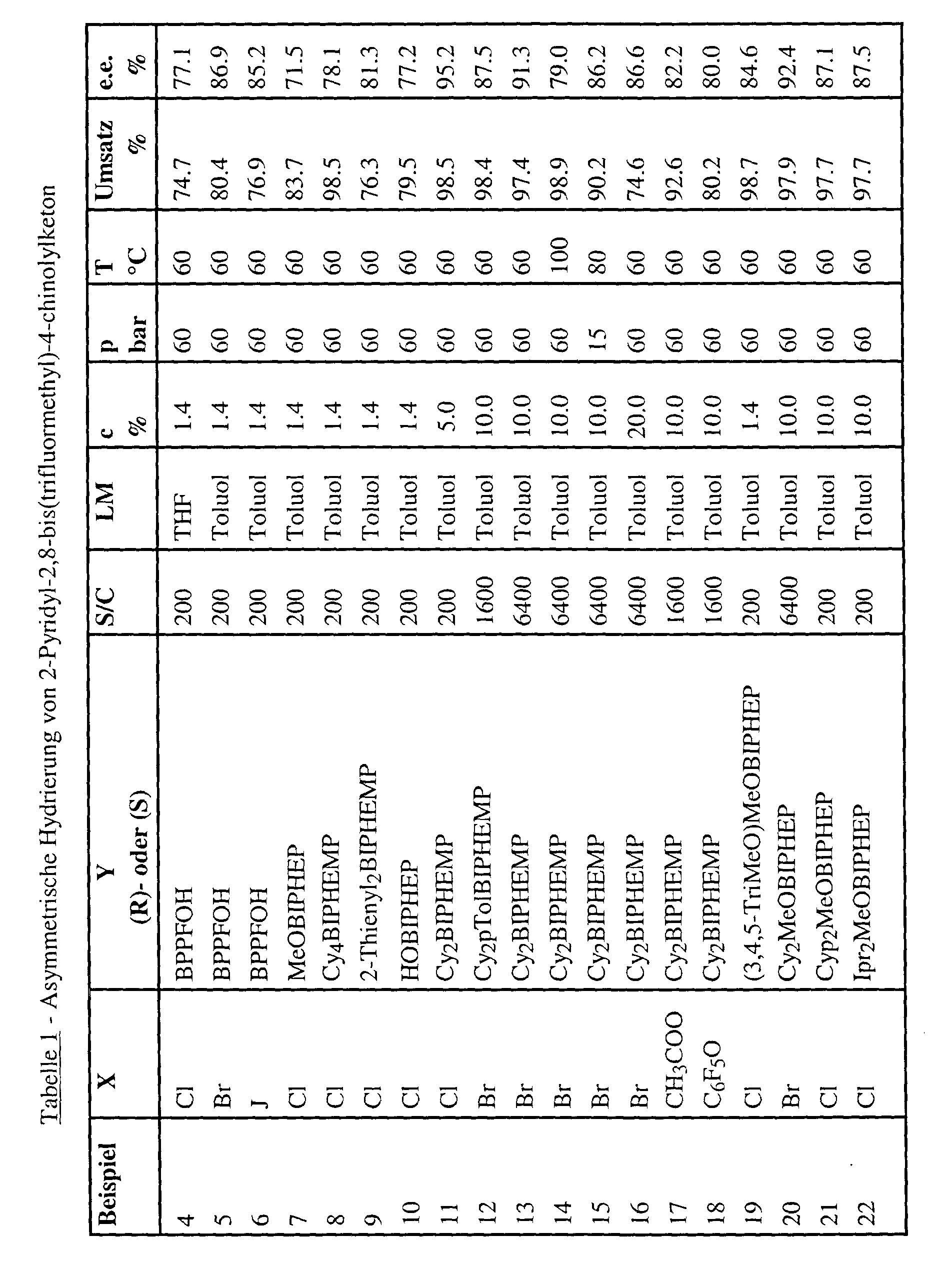

### Beispiel 23

Das gemäss vorhergehenden Beispielen verwendete (R)- oder (S)-P,P-Dicyclohexyl-P',P'-diphenyl-(6,6'-dimethylbiphenyl-2,2'-diyl)diphosphin wurde wie folgt hergestellt:
a) Zu einer auf -76° gekühlten Lösung von 20,0 g (0,046 Mol) (R)-2,2'-Dijod-6,6'-dimethylbiphenyl (T. Frejd and T. Klingstedt, J. Chem. Soc., Chem. Commun. (1983) 1021), gelöst in 430 ml absolutem Toluol und 90 ml Aether wurden unter Ar-Begasung 32 ml einer tert. Butyllithium-Lösung (1,6M in Pentan; 0,051 Mol) zugegeben und das Gemisch 3/4 Stunden bei -75° gerührt. Aus einem Tropftrichter wurden anschliessend innert 1/4 Stunde eine Lösung von 20,1 g (0,092 Mol) Chlordiphenylphosphin in 100 ml absolutem Toluol zugetropft, das Gemisch 1 Stunde bei -70°, und nach dem Entfernen des Kühlbades über Nacht bei Raumtemperatur gerührt. Zur Aufarbeitung wurde das Reaktionsgemisch, eine grau-beige Suspension, mit 150 ml Wasser versetzt, mit 80 ml 3N NaOH alkalisch gestellt und mit 300 ml Toluol extrahiert. Die organische Phase wurde 2 x mit je 200 ml Wasser neutral gewaschen, getrocknet (Na₂SO₄), eingedampft und der resultierende Rückstand (32,5 g; gelbes Oel) an 450 g Kieselgel chromatographiert. In drei Hauptfraktionen eluierte man: mit 4,5 l Hexan 3,2 g unverändertes Ausgangsmaterial, mit 15 l Hexan-Toluol (95:5)-Gemisch 13,1 g (71,3%) enantiomerenreines Monojodid als weisse Kristalle, und mit 2 l Toluol 1,5 g weisses kristallines (R)-BIPHEMP (GC-Gehalt: 75%). Zur Analyse wurde das Monojodid (13,1 g) aus Aethylacetat/Methanol umkristallisiert: (R)-Diphenyl-(2'-jodo-6,6'-dimethylbiphenyl-2-yl)phosphin; Smp.: 167,5-168,4°; [α]_{D},²⁰ -44,8 (c=1; CHCl₃).
   Auf analoge Weise wurde hergestellt:
   (S)-Diphenyl-(2'-jod-6,6'-dimethylbiphenyl-2-yl)phosphin; Smp.: 167,5-168,7°; [α]_{D},²⁰ +43,5 (c=1; CHCl₃).
b) In einem Gemisch aus 100 ml absolutem Toluol, 200 ml Aether und 0,5 ml Triäthylamin, wurden unter Ar-Begasung 8,0 g (0,0162 Mol) (S)-Diphenyl-(2'-jod-6,6'-dimethylbiphenyl-2-yl)phosphin gelöst, auf -65° abgekühlt, 12 ml tert. Butyllithium-Lösung (1,6M in Pentan; 0,019 Mol) zugegeben und das Reaktionsgemisch 1 Stunde bei-60° gerührt. Anschliessend wurden auf einmal 5,0 g (0,021 Mol)
   Dicyclohexylchlorphosphin zugegeben, mit wenig Toluol nachgespült und die grau-beige Suspension über Nacht bei Raumtemperatur gerührt. Zur Aufarbeitung wurde das Gemisch mit 50 ml Wasser und 50 ml 3N NaOH versetzt, 1/4 Stunde gerührt, mit 300 ml Toluol extrahiert, die organische Phase einmal mit 200 ml Wasser gewaschen, getrocknet (Na₂SO₄), filtriert und eingedampft. Nach der Umkristallisation des Rohproduktes [12 g; (S)-Cy₂BIPHEMP; GC-Gehalt: 87%] aus Aethylacetat, resultierten 6,2 g (68%) (S)-P,P-Dicyclohexyl-P',P'-diphenyl-(6,6'-dimethylbiphenyl-2,2'-diyl)diphosphin als weisse Kristalle; Smp.: 185,4° (99,8% e.e., gemäss HPLC-Analyse an einer Chircacel OD-Phase; GC-Gehalt: 99,2%); [α]_{D},²⁰ +52,3 (c=1; CHCl₃).

Auf analoge Weise wurde hergestellt:
(R)-P,P-Dicyclohexyl-P',P'-diphenyl-(6,6'-dimethylbiphenyl-2,2'-diyl)diphosphin; Smp.: 186-186,9°; (99,8% e.e.; GC: 99,3%): [α]_{D},²⁰ -52,8 (c=1; CHCl₃).

### Beispiel 24

In zu Beispiel 23 analoger Weise wurden folgende, in den Beispielen 9 und 12 verwendeten Diphosphine hergestellt:
(R)-Di-p-tolyl-(2'-jod-6,6'-dimethylbiphenyl-2-yl)phosphin; Smp.: 135-136°; [α]_{D},²⁰ -55,1 (c=1,0, CHCl₃).
(S)-Di-p-tolyl-(2'-jod-6,6'-dimethylbiphenyl-2-yl)phosphin; Smp.: 136,4°; [α]_{D},²⁰ +55,7 (c=1,0, CHCl₃).
(R)-P,P-Dicyclohexyl-P',P'-di-p-tolyl-(6,6'-dimethylbiphenyl-2,2'-diyl)diphosphin; Smp.: 167°; (98,6% e.e.; GC: 98%); [α]_{D},²⁰ -71,2 (c=1; CHCl₃).
(S)-P,P-Dicyclohexyl-P',P'-di-p-tolyl-(6,6'-dimethylbiphenyl-2,2'-diyl)diphosphin; Smp.: 166,1°; (97% e.e.; GC: 98%); [α]_{D},²⁰ +69,4 (c=1; CHCl₃).
(R)-P,P-Diphenyl-P',P'-di-2-thienyl-(6,6'-dimethylbiphenyl-2,2'-diyl)diphosphin; Smp.: 151-151,3°; (99,2% e.e.; GC: 99,5%); [α]_{D},²⁰ +71,9 (c=1; CHCl₃).
(S)-P,P-Diphenyl-P',P'-di-2-thienyl-(6,6'-dimethylbiphenyl-2,2'-diyl)diphosphin; Smp.: 149-150°; (99,9% e.e.; GC: 99,7%); [α]_{D},²⁰ -70,2 (c=1; CHCl₃).

### Beispiel 25

Das gemäss Beispiel 10 eingesetzte (R)- bzw. (S)-HOBIPHEP wurde wie folgt hergestellt.

Eine Lösung von 11,64 g (19,98 mMol) (R)-(6,6'-Dimethoxybiphenyl-2,2'-diyl)bis(diphenylphosphin)[= (R)-MeOBIPHEP) in 200 ml CH₂Cl₂ (über Molekularsieb getrocknet) wurde unter Argon auf -78° abgekühlt. Eine Lösung von 50 ml (50 mMol) 1M BBr₃ in CH₂Cl₂ wurde dann langsam zugefügt, wobei gegen Ende der Zugabe ein weisser Niederschlag entstand. Das Gemisch wurde sodann auf Raumtemperatur aufgewärmt und während 24 Stunden gerührt. Nach Hydrolyse mit 400 ml gesättigtem NH₄Cl während 1 Stunde, Phasentrennung, Trocknen der organischen Phase (CaCl₂) und Eindampfen erhielt man 12,5 g eines gelben Pulvers. Nach Chromatographie an Silicagel (90 g, Hexan/CH₂Cl₂ 1:1, Hexan/Aethylacetat 9:1 ⌀ 3:2) erhielt man 8,26 g (R)-HOBIPHEP als weisses Pulver. Smp. 198,2°-199,4°. [α]_{D},²⁰ = -34,7° (c = 0,9, CHCl₃).

Die Umsetzung von 0,65 g (1,1 mMol) (S)-MeOBIPHEP mit 2,2 ml (2,2 mMol) 1M BBr₃ in CH₂Cl₂ in zum Vorherigen analoger Weise ergab 0,35 g (S)-HOBIPHEP als weisses Pulver. Smp. 199,5°-200,5°. [α]_{D},²⁰ = +33,9° (c = 1, CHCl₃).

### Beispiel 26

Das gemäss Beispiel 20 verwendete (R)- oder (S)-P,P-Dicyclohexyl-P',P'-diphenyl-(6,6'-dimethoxybiphenyl-2,2'-diyl)diphosphin wurde wie folgt hergestellt:
a) In einem Gemisch aus 150 ml absolutem Toluol und 50 ml Aether wurden unter Argon-Begasung 10,2 g (0,019 Mol) (R)-Diphenyl-(2'-jodo-6,6'-dimethoxybiphenyl-2-yl)phosphin gelöst, die Lösung auf -70° abgekühlt, dann mit 15 ml Butyllithium-Lösung (1,6M in Hexan; 0,023 Mol) versetzt und das Reaktionsgemisch 45 Minuten bei -69° gerührt. Anschliessend wurden bei -65° innert 15 Minuten 10,0 g (0,043 Mol) Dicyclohexylchlorphosphin, gelöst in 50 ml Toluol, zugetropft, und die grau-beige Suspension 1 Stunde bei -65°, dann über Nacht bei Raumtemperatur gerührt. Zur Aufarbeitung wurde das Gemisch mit 85 ml Wasser und 15 ml 3N NaOH versetzt, 15 Minuten gerührt, mit 300 ml Toluol extrahiert, die organische Phase zweimal mit 150 ml Wasser gewaschen, getrocknet (Na₂SO₄), filtriert und eingedampft. Nach einer chromatographischen Filtration an 400 g Kieselgel (Hexan/Toluol 1:1) und Umkristallisation des Rohproduktes aus Aethylacetat, erhielt man 4,7 g (R)-P,P-Dicyclohexyl-P',P'-diphenyl-(6,6'-dimethoxybiphenyl-2,2'-diyl)diphosphin [(R)-Cy₂MeOBIPHEP] als weisse Kristalle. Smp. 239,3°; [α]_{D},²⁰ = +10,3 (c=1; CHCl₃).
   Auf analoge Weise wurde hergestellt:
   (S)-P,P-Dicyclohexyl-P',P'-diphenyl-(6,6'-dimethoxybiphenyl-2,2'-diyl)diphosphin, [(S)-Cy₂MeOBIPHEP].
b) Die als Ausgangsmaterial verwendeten (R)- und (S)-Diphenyl-(2'-jodo-6,6'-dimethoxy-1,1'-biphenyl-2-yl)phosphine wurden wie folgt hergestellt:
   Zu einer auf -76° gekühlten Lösung von 13,7 g (0,029 Mol) (S)-2,2'-Dijodo-6,6'-dimethoxy-1,1'-biphenyl, gelöst in 200 ml absolutem Toluol und 50 ml Aether wurden unter Argon-Begasung 18 ml einer tert.-Butyllithium-Lösung (15%ige Lösung in Pentan; 0,028 Mol) zugegeben und das Gemisch 1 Stunde bei -70° gerührt. Aus einem Tropftrichter wurde anschliessend innert 15 Minuten eine Lösung von 13 g (0,062 Mol) Chlordiphenylphosphin in 50 ml absolutem Toluol zugetropft, das Gemisch 1 Stunde bei-70°, und nach dem Entfernen des Kühlbades 1 Stunde bei Raumtemperatur gerührt. Zur Aufarbeitung wurde das Reaktionsgemisch mit 70 ml Wasser versetzt, mit 30 ml 3N NaOH alkalisch gestellt und mit 500 ml Essigester extrahiert. Die organische Phase wurde mit 150 ml Wasser neutral gewaschen, getrocknet (Na₂SO₄), eingedampft und der resultierende Rückstand an 300 g Kieselgel chromatographiert. In zwei Hauptfraktionen eluierte man: mit 2,1 l Hexan/Toluol (6:4)-Gemisch 4,0 g unverändertes Ausgangsmaterial und mit 3,5 l Hexan/Toluol (1:1)-Gemisch 9,9 g enantiomerenreines Monojodid, als weisse Kristalle, die direkt in die nächstfolgende Stufe eingesetzt wurden. Aus den polareren Fraktionen konnten noch ca. 120 mg kristallines (S)-MeOBIPHEP isoliert werden. Zur Analyse wurde eine Probe des Monojodids aus Aethylacetat/Methanol umkristallisiert:
   (S)-Diphenyl-(2'-jodo-6,6'-dimethoxy-1,1'-biphenyl-2-yl)phosphin; Smp.: 125,7°; [α]_{D},²⁰ = -9,0 (c=0,7; CHCl₃).

   Auf analoge Weise wurde hergestellt:
   (R)-Diphenyl-(2'-jodo-6,6'-dimethoxy-1,1'-biphenyl-2-yl)phospin;
   (R,S)-Diphenyl-(2'-jodo-6,6'-dimethoxy-1,1'-biphenyl-2-yl)phosphin; Smp.: 194,0-194,4°.
c) Die gemäss b) als Ausgangsmaterial verwendeten (R)- und (S)-2,2'-Dijod-6,6'-dimethoxy-1,1'-biphenyle wurden wie folgt hergestellt:
   12,15 g (0,05 Mol) (S)-2,2'-Diamino-6,6'-dimethoxy-1,1'-biphenyl wurden in einem Gemisch aus 288 ml Wasser und 100 ml konz. Schwefelsäure bei ca. 30°-40° gelöst und die Lösung in einem Kühlbad auf 2° abgekühlt. Zu diesem Gemisch wurde innert 30 Minuten bei 2° eine Lösung aus 9,6 g (0,140 Mol) Natriumnitrit in 25 ml Wasser zugetropft und nach beendeter Zugabe 30 Minuten bei 4° gerührt. Anschliessend wurde mit 150 ml Toluol versetzt, aus einem Tropftrichter innert 5 Minuten eine Lösung aus 42 g (0,253 Mol) Kaliumjodid und 16,8 g (0,1 Mol) Jod in 75 ml Wasser zugetropft und 3 Stunden bei Raumtemperatur gerührt. Zur Aufarbeitung wurde das Reaktionsgemisch mit 300 ml Aethylacetat und 200 ml Toluol extrahiert, die organischen Phasen 2 x mit je 200 ml einer 10%igen wässrigen Natriumthiosulfatlösung und 2 x mit je 150 ml Wasser gewaschen, vereinigt, getrocknet (Na₂SO₄), eingedampft und das resultierende Rohprodukt an 150 g Kieselgel chromatographiert. Mit 10 l Hexan/Dichlormethan-(4:1 bis 1:1)-Gemisch wurde eluiert, einmal aus Hexan umkristallisiert und man erhielt 14,2 g weisses kristallines enantiomerenreines (S)-2,2'-Dijod-6,6'-dimethoxy-1,1'-biphenyl. Smp.: 163,5°; [α]_{D},²⁰ = -55,1° (c=1; CHCl₃).
   Auf analoge Weise wurde hergestellt:
   (R)-2,2'-Dijod-6,6'-dimethoxy-1,1'-biphenyl. Smp.: 152°; [α]_{D},²⁰ = +54,4° (c=1; CHCl₃).
d) Die gemäss c) wiederum als Ausgangsmaterial verwendeten (R)- und (S)-2,2'-Diamino-6,6'-dimethoxy-1,1'-biphenyle wurden wie folgt hergestellt:
   80 g (0,33 Mol) (rac)-2,2'-Diamino-6,6'-dimethoxy-1,1'-biphenyl wurden in 600 ml Aethylacetat und 150 g (SS)-Di(phenylaminocarbonyloxy)-bernsteinsäure [(SS)-DIPACOSA] in einem Gemisch aus 400 ml Aethylacetat und 50 ml Methanol bei ca. 60° gelöst, die beiden Lösungen bei 60° vereinigt und zur Kristallisation über Nacht bei Raumtemperatur stehen gelassen. Nach dem Abnutschen, Waschen (150 ml Aethylacetat) und Trocknen (14 mm Hg; Raumtemperatur; 30 Minuten) des weissen Kristallisats wurden 173,9 g (S)-2,2'-Diamino-6,6'-dimethoxy-1,1'-biphenyl-(SS)-DIPACOSA (1:2)-Assoziat erhalten. [(S)/(R)-Gehalt: 99%/1%].

Zur Verbesserung der Enantiomerentrennung wurde das Kristallisat (173,9 g) in 300 ml Aethylacetat aufgeschlämmt, für 15 Minuten bei 60° gerührt, die Suspension über Nacht bei Raumtemperatur stehen gelassen, abgenutscht, der Rückstand mit Aethylacetat (100 ml) gewaschen, getrocknet (14 mm Hg / Raumtemperatur) und auf diese Weise 154 g (1:2)-Assoziat erhalten [(S)/(R)-Gehalt: 99,7%/0,3%].
Zur Abtrennung des Spaltreagens wurde dieses Material (154 g) in 2 l Aethylacetat gelöst, 700 ml Wasser hinzugefügt und unter Rühren die Wasserphase mit einer ges.
Natriumbicarbonat-Lösung auf pH 8 gestellt. Nach dem Abtrennen der organischen Phase, die gewaschen (200 ml Wasser), getrocknet (Na₂SO₄) und eingedampft wurde, resultierten 30,9 g öliges (S)-2,2'-Diamino-6,6'-dimethoxy-1,1'-biphenyl [(S)/(R)-Gehalt: 99,8% / 0,2%]. [α]_{D},²⁰ = -32,0° (c=1; CHCl₃).

Auf analoge Weise wurde, ausgehend von racemischem Diamin mit (RR)-DIPACOSA, das ölige (R)-2,2'-Diamino-6,6'-dimethoxy-1,1'-biphenyl gewonnen, das aus Diäthyläther umkristallisiert werden konnte [(S)/(R)-Gehalt: 0% / 100%]. Smp.: 86,9°; [α]_{D},²⁰ = +32,8° (c=1; CHCl₃).

In zum vorhergehenden analoger Weise wurden auch folgende, in den Beispielen 21 und 22 verwendeten Diphosphine hergestellt:
(S)-P,P-Dicyclopentyl-P',P'-diphenyl-(6,6'-dimethoxybiphenyl-2,2'-diyl)diphosphin; [(S)-Cyp₂MeOBIPHEP]. Smp. 210,9°; [α]_{D},²⁰ = -15,7° (c=0,4; CHCl₃).
(R)-P,P-Dicyclopentyl-P',P'-diphenyl-(6,6'-dimethoxybiphenyl-2,2'-diyl)diphosphin; [(R)-Cyp₂MeOBIPHEP].
(S)-P,P-Diisopropyl-P',P',diphenyl-(6,6'-dimethoxybiphenyl-2,2'-diyl)diphosphin; [(S)-Ipr₂MeOBIPHEP]. Smp. 147,9°; [α]_{D},²⁰ = -29° (c=0,5; CHCl₃).
(R)-P,P-Diisopropyl-P',P'-diphenyl-(6,6'-dimethoxybiphenyl-2,2'-diyl)diphosphin; [(R)-Ipr₂MeOBIPHEP].

### Beispiel 27

In einem 5 1-Hydrierkolben wurden 18 g Platinoxid und 18 g Aktivkohle in 500 ml Methanol während 1 Stunde unter Normalbedingungen vorhydriert. Anschliessend wurden eine Lösung von 185,0 g (0,50 Mol) (S)-α-(2-pyridyl)-2,8-bis(trifluormethyl)-4-chinolinmethanol (>99% e.e.) und 60 ml 37% Salzsäure in 1300 ml Methanol zugegeben. Die Hydrierung wurde bei 40° unter Normaldruck während 8 Stunden durchgeführt, wobei 37 l Wasserstoff aufgenommen wurden. Der Umsatz betrug gemäss DC 100%. Nach dem Abkühlen wurde der Katalysator abfiltriert, mit Methanol gewaschen und das Filtrat eingedampft. Der gelbe, kristalline Rückstand (210 g Hydrochlorid) bestand gemäss GC aus 85% des erythro- und 15% des entsprechenden threo-Isomeren. Die e.e.-Werte beider Diastereomeren betrugen mindestens 98% (GC einer silylierten Probe).

50 g des rohen Hydrochlorids wurden durch Verteilung zwischen 150 ml 1N Natronlauge und 0,5 l Aether in das entsprechende Gemisch der erythro- und threo-Base übergeführt (43 g), welches zur Trennung der Isomeren an 1 kg Kieselgel chromatographiert wurde. Mit einem mit 25% Ammoniumhydroxid gesättigten (20:1)-Gemisch von Methylenchlorid und Methanol als Eluiermittel wurden neben Mischfraktionen 4,2 g eines threo-Isomeren und 19 g reines erythro-Isomeres erhalten.

Zur Analyse wurden die beiden Isomeren aus Methanol-Toluol umkristallisiert.
(S)-α-[(S)-2-Piperidinyl]-2,8-bis(trifluormethyl)-4-chinolinmethanol (threo); weisse Kristalle; Smp. 175-176°; [α]_{D},²⁰ -21,8 (c=0,4, Methanol).
(S)-α-[(R)-2-Piperidinyl]-2,8-bis(trifluormethyl)-4-chinolinmethanol (erythro); weisse Kristalle; Smp. 170,5-171,0°; [α]_{D},²⁰ -33,8 (c=0,4, Methanol).

In analoger Weise wurden hergestellt:
(R)-α-[(R)-2-Piperidinyl]-2,8-bis(trifluormethyl)-4-chinolinmethanol (threo); Smp. 176-177°; [α]_{D},²⁰ +22,0 (c=0,4, Methanol).
(R)-α-[(S)-2-Piperidinyl]-2,8-bis(trifluormethyl)-4-chinolinmethanol (erythro); Smp. 170,5-171,0°; [α]_{D},²⁰ +35,5 (c=0,4, Methanol).

## Patentansprüche

1. Verfahren zur Herstellung der Verbindung der Formel 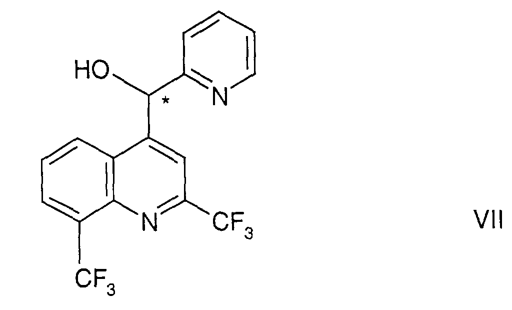 **dadurch gekennzeichnet, dass** man das Keton der Formel 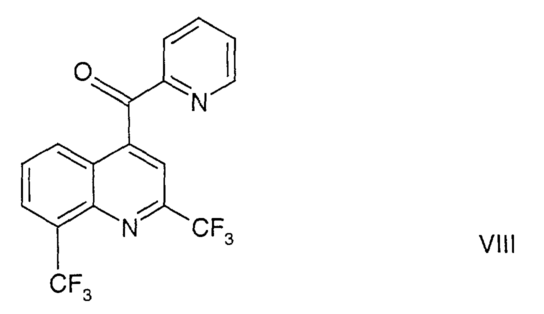 asymmetrisch hydriert, und zwar
in Gegenwart eines Rhodium-Diphosphin-Komplexes der Formel
[Rh(X)(Y)(L_{0,1,2}]_{1,2} III
worin X, Y und L folgende Bedeutung haben:
X= Halogenid, Z-COO-, Phenolat oder halogeniertes Phenolat;
Z= C₁-C₄-Alkyl, unsubstituiertes Phenyl, halogeniertes C₁-C₄-Alkyl oder halogeniertes Phenyl;
Y = chiraler, atropisomerer Diphosphinligand der Formel 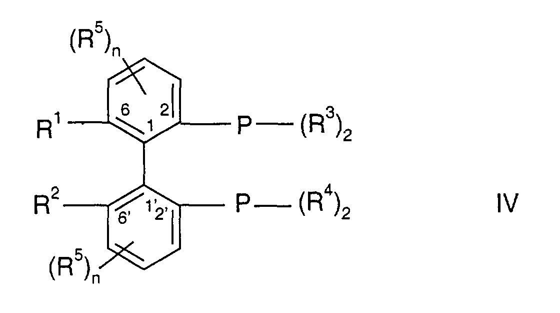
oder der Formel 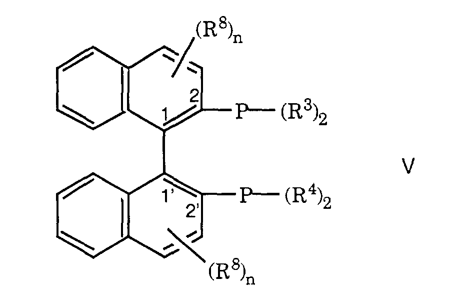 oder ein Ferrocenyl-Diphosphin der Formel 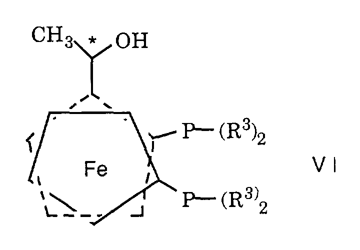 mit:
R¹,R² = unabhängig voneinander C₁-C₄-Alkyl, C₁-C₄-Alkoxy, Di-C₁-C₄-Alkylamino, Hydroxy, durch Benzyl, Allyl, Benzyloxymethyl, C₁-C₄-Alkoxymethyl oder 2-Methoxyäthoxymethyl geschütztes Hydroxy oder Hydroxymethyl, Hydroxymethyl, oder R¹ und R² zusammen die Gruppen
(―CH₂―)ₘ, ―CH₂―O―CH₂―, 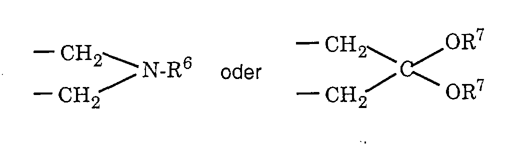
m= Zahl 3, 4, 5,
R⁶ = C₁-C₄-Alkyl oder unsubstituiertes oder einfach oder mehrfach durch Phenyl, C₁-C₄-Alkyl, C₁-C₄-Alkoxy, di-C₁-C₄-Alkylamino, Fluor oder Tri-C₁-C₄-Alkylsilil substituiertes Phenyl oder Benzyl,
R⁷ = C₁-C₄-Alkyl oder beide R⁷ zusammen Di- oder Trimethylen,
R³,R⁴ = unabhängig voneinander C₁-C₄-Alkyl, oder unsubstituiertes oder einfach oder mehrfach durch Phenyl, C₁-C₄-Alkyl, C₁-C₄-Alkoxy, di-C₁-C₄-Alkylamino, Fluor oder Tri-C₁-C₄-Alkylsilil substituiertes Phenyl, C₃-C₇-Cycloalkyl, einen fünfgliedrigen Heteroaromaten der Formeln (a), (b), (c), oder (d) 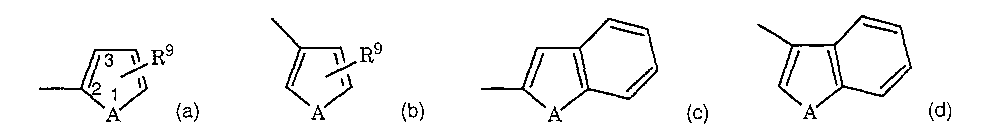 worin A Sauerstoff, Schwefel oder NR¹⁰, R⁹ Wasserstoff, C₁-C₄-Alkyl, C₁-C₄-Alkoxy und R¹⁰ C₁-C₄-Alkyl, bedeutet
oder eine Gruppe der Formel 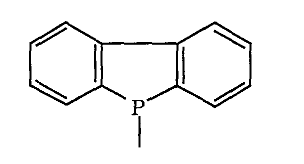
R⁵ = C₁-C₄-Alkyl, C₁-C₄-Alkoxy,
R⁸ = bevorzugt in 5,5'-Position, Methyl, Aethyl, Halogen, -OH, -NH₂, Acetylamino, -NO₂, -SO₃H,
n = Zahl 0, 1, 2, 3, und
L= neutraler Ligand, ausgewählt aus einer Gruppe von Olefinen, bestehend aus Ethylen, Propylen, Cycloocten, 1,5-Hexadien, Norbornadien, 1,5-Cyclooctadien oder von Nitrilen, bestehend aus Acetonitril oder Benzonitril oder von den verwendeten Lösungsmitteln.

2. Verfahren gemäss Anspruch 1, **dadurch gekennzeichnet, dass** man die asymmetrische Hydrierung durchführt unter Verwendung eines Katalysators der Formel III, worin X Chlor oder Brom, vorzugsweise Brom, bedeutet und Y einen chiralen, atropisomeren Liganden der Formel IV bedeutet, worin R¹ und R² gleich sind und Methyl oder Methoxy darstellen, n = 0 ist und R³ und R⁴ voneinander verschieden sind und Phenyl bzw. Cyclohexyl darstellen.

3. Verfahren gemäss Anspruch 1 oder 2, **dadurch gekennzeichnet, dass** man die asymmetrische Hydrierung bei Temperaturen von etwa 20°C bis etwa 140°C und unter einem Druck von etwa 1 bis 100 bar durchführt.

## Claims

1. A process for the manufacture of the compound of the formula 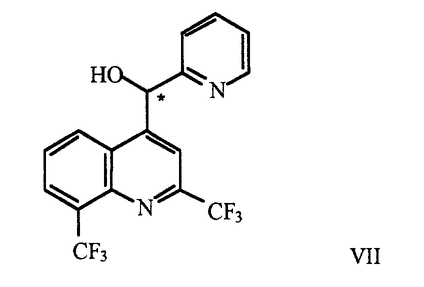 **characterized by** asymmetrically hydrogenating the ketone of the formula 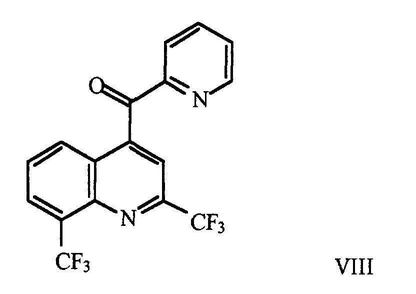 in the presence of a rhodium-diphosphine complex of the formula
[Rh(X)(Y)(L_{0,1,2}]_{1,2} III
wherein X, Y and L have the following significance:
X= halogenide, Z-COO-, phenolate or halogenated phenolate;
Z = C₁-C₄-alkyl, unsubstituted phenyl, halogenated C₁-C₄-alkyl or halogenated phenyl;
Y= chiral, atropisomeric diphosphine ligand of the formula 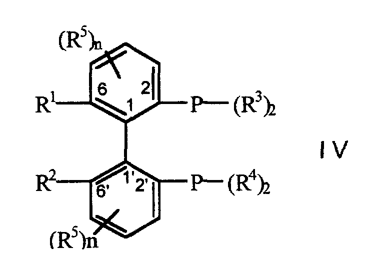 or the formula 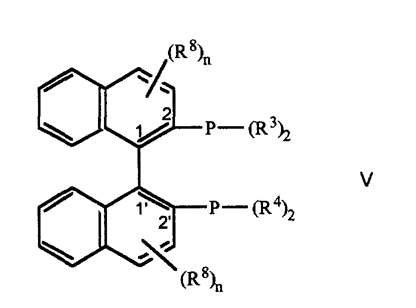 or a ferrocenyl-diphosphine of the formula 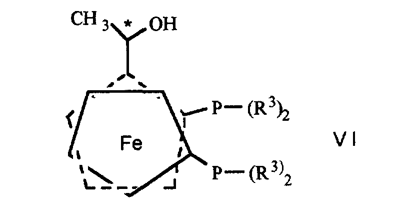 with:
R¹, R² = independently of each other C₁-C₄-alkyl, C₁-C₄-alkoxy, di-C₁-C₄-alkylamino, hydroxy, hydroxy or hydroxymethyl protected by benzyl, allyl, benzyloxymethyl, C₁-C₄-alkoxymethyl or 2-methoxyethoxymethyl, hydroxymethyl, or R¹ and R² together the groups
(―CH₂―)ₘ, ―CH₂―O―CH₂―, 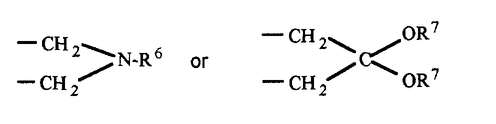
m = integer 3, 4, 5,
R⁶ = C₁-C₄-alkyl, or phenyl or benzyl unsubstituted or substituted by one or more phenyl, C₁-C₄-alkyl, C₁-C₄-alkoxy, di-C₁-C₄-alkylamino, fluorine or tri-C₁-C₄-alkylsilyl,
R⁷ = C₁-C₄-alkyl or both R^{7'}s together di- or trimethylene,
R³, R⁴ = independently of each other C₁-C₄-alkyl, or phenyl unsubstituted or substituted by one or more phenyl, C₁-C₄-alkyl, C₁-C₄-alkoxy, di-C₁-C₄-alkylamino, fluorine or tri-C₁-C₄-alkylsilyl, C₃-C₇-cycloalkyl, a five-membered heteroaromatic of formulae (a), (b), (c), or (d) 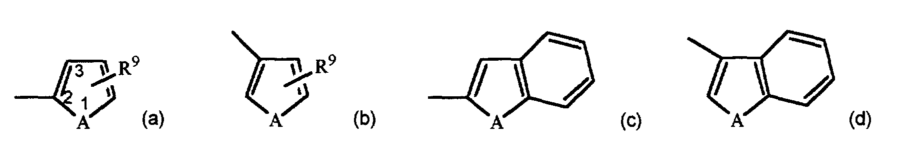 wherein A signifies oxygen, sulphur or NR¹⁰, R⁹ signifies hydrogen, C₁-C₄-alkyl, C₁-C₄-alkoxy and R¹⁰ signifies C₁-C₄-alkyl,
or a group of the formula 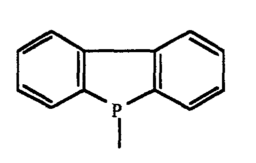
R⁵ = C₁-C₄-alkyl, C₁-C₄-alkoxy,
R⁸ = methyl, ethyl, halogen -OH, -NH₂, acetylamino, -NO₂, -SO₃H, preferably in the 5,5'-position,
n= integer 0, 1, 2, 3, and
L= neutral ligand selected from a group of olefins consisting of ethylene, propylene, cyclooctene, 1,5-hexadiene, norbornadiene, 1,5-cyclooctadiene or of nitriles consisting of acetonitrile or benzonitrile or of the solvents used.

2. A process according to claim 1, **characterized in that** the asymmetric hydrogenation is carried out using a catalyst of formula III in which X signifies chlorine or bromine, preferably bromine, and Y signifies a chiral, atropisomeric ligand of formula IV in which R¹ and R² are identical and represent methyl or methoxy, n = 0 and R³ and R⁴ are different from each other and represent phenyl or cyclohexyl.

3. A process according to claim 1 or 2, **characterized in that** the asymmetric hydrogenation is carried out at temperatures of about 20°C to about 140°C and under a pressure of about 1 to 100 bar.

## Revendications

1. Procédé pour la préparation du composé de formule 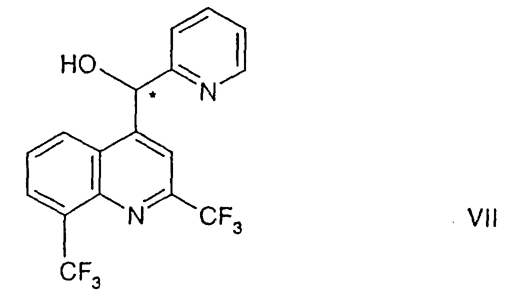 **caractérisé en ce qu'**on soumet à une hydrogénation asymétrique la cétone de formule 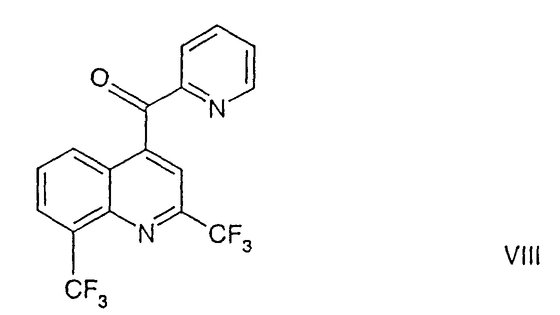 à savoir
en présence d'un complexe rhodium-diphosphine de formule
[Rh(X)(Y)(L_{0,1,2})]_{1,2} III
dans laquelle X, Y et L ont les significations suivantes :
X = halogénure, un groupe Z-COO-, phénolate ou phénolate halogène ;
Z = un groupe alkyle en C₁-C₄, phényle non substitué, alkyle en C₁-C₄, halogéné ou phényle halogène ;
Y = un ligand diphosphine chiral, atropisomère, de formule 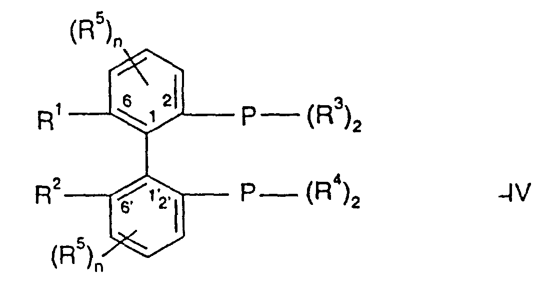 ou de formule 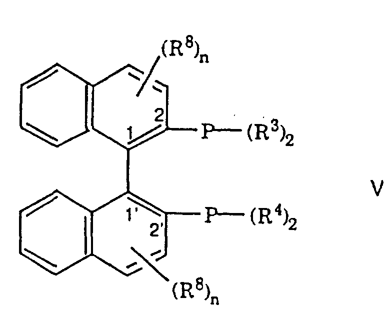 ou une ferrocényldiphosphine de formule 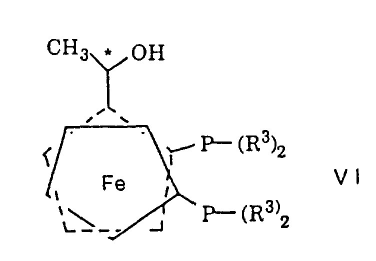 où
R¹, R² représentent, indépendamment l'un de l'autre, un groupe alkyle en C₁-C₄, alcoxy en C₁-C₄, dialkyl(C₁-C₄)amino, hydroxy, un groupe hydroxy ou hydroxyméthyle protégé par un groupe benzyle, allyle, benzyloxyméthyle, alcoxy(C₁-C₄)méthyle ou 2-méthoxyéthoxyméthyle, ou le groupe hydroxyméthyle, ou R¹ et R² forment ensemble les groupes
(-CH₂-)ₘ, -CH₂-O-CH₂-, 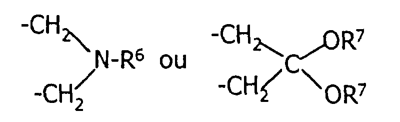
m= le nombre 3, 4, 5
R⁶ représente un groupe alkyle en C₁-C₄ ou un groupe phényle ou benzyle non substitué ou substitué une ou plusieurs fois par un ou plusieurs atomes de fluor ou groupes phényle, alkyle en C₁-C₄, alcoxy en C₁-C₄, dialkyl(C₁-C₄)amino ou trialkyl(C₁-C₄)silyle,
R⁷ représente un groupe alkyle en C₁-C₄, ou les deux groupes R⁷ forment ensemble un groupe di- ou triméthylène,
R³, R⁴ représentent, indépendamment l'un de l'autre, un groupe alkyle en C₁-C₄, ou un groupe phényle non substitué ou substitué une ou plusieurs fois par un ou plusieurs atomes de fluor ou groupes phényle, alkyle en C₁-C₄, alcoxy en C₁-C₄, dialkyl(C₁-C₄)amino ou trialkyl(C₁-C₄)silyle, ou un groupe cycloalkyle en C₃-C₇, un groupe hétéroaromatique à cinq chaînons de formule (a), (b), (c) ou (d) 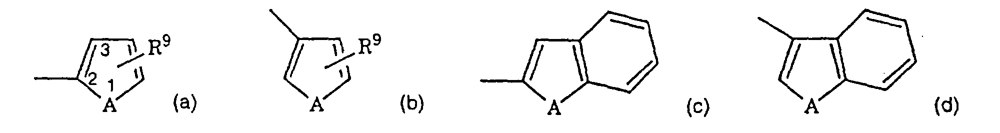 formules dans lesquelles A représente un atome d'oxygène ou de soufre, ou un groupe NR¹⁰, R⁹ représente un atome d'hydrogène ou un groupe alkyle en C₁-C₄, alcoxy en C₁-C₄, et R¹⁰ représente un groupe alkyle en C₁-C₄,
ou un groupe de formule 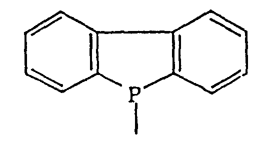
R⁵ = un groupe alkyle en C₁-C₄, alcoxy en C₁-C₄,
R⁸ = de préférence en position 5,5', un atome d'halogène, le groupe méthyle, éthyle, -OH, -NH₂, acétylamino, -NO₂, -SO₃H,
n = le nombre 0, 1, 2, 3 et
L = un ligand neutre choisi dans un ensemble d'oléfines constitué par l'éthylène, le propylène, le cyclo-octène, le 1,5-hexadiène, le norbornadiène, le 1,5-cyclo-octadiène ou les nitriles consistant en l'acétonitrile et le benzo-nitrile, ou par les solvants utilisés.

2. Procédé selon la revendication 1, **caractérisé en ce qu'**on effectue l'hydrogénation asymétrique en utilisant un catalyseur de formule III, dans laquelle X représente le chlore ou le brome, de préférence le brome, et Y représente un ligand chiral atropisomère de formule IV, dans laquelle R¹ et R² sont identiques et représentent le groupe méthyle ou méthoxy, n = 0, et R³ et R⁴ sont différents l'un de l'autre et représentent le groupe phényle ou cyclohexyle.

3. Procédé selon la revendication 1 ou 2, **caractérisé en ce qu'**on effectue l'hydrogénation asymétrique à des températures d'environ 20°C à environ 140°C et sous une pression d'environ 1 à 100 bars.
